(19) Europäisches Patentamt
European Patent Office
Office européen des brevets

(11) **EP 3 542 718 A1**

(12) **EUROPEAN PATENT APPLICATION**
published in accordance with Art. 153(4) EPC

(43) Date of publication:
**25.09.2019  Bulletin 2019/39**

(21) Application number: **17872688.1**

(22) Date of filing: **15.11.2017**

(51) Int Cl.:
***A61B 5/091*** (2006.01)     ***A61B 5/053*** (2006.01)

(86) International application number:
**PCT/KR2017/012920**

(87) International publication number:
**WO 2018/093133 (24.05.2018 Gazette 2018/21)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **18.11.2016  KR 20160154142**

(71) Applicant: **Bilab Co., Ltd.**
**Seoul 06779 (KR)**

(72) Inventors:
• **WOO, Eung Je**
**Seongnam-si**
**Gyeonggi-do 13633 (KR)**
• **OH, Tong In**
**Hwaseong-si**
**Gyeonggi-do 18475 (KR)**

(74) Representative: **Santarelli**
**49, avenue des Champs-Elysées**
**75008 Paris (FR)**

(54) **DEVICE FOR PULMONARY FUNCTION TESTING AND METHOD THEREFOR**

(57)     Disclosed are a method and apparatus for a pulmonary function test, in which a pulmonary function is diagnosed based on vital capacity according to a breathing parameter of a subject and a thorax image according to impedance data, in which a conventional spirometer and electrical impedance tomography (EIT) are used together to collectively process and analyze a global variable obtained at a mouth of a subject and a local variable obtained from an image of an interior of a lung, thereby proposing spirotomometry.

FIG. 1

**Description**

[TECHNICAL FIELD]

The disclosure relates to a method and apparatus for a pulmonary function test, and more particularly to a method and apparatus for a pulmonary function test, in which a pulmonary function is diagnosed based on vital capacity according to a breathing parameter of a subject and a thorax image according to impedance data.

[BACKGROUND ART]

A diagnostic spirometer refers to a medical device for diagnosing a pulmonary function of a subject by measuring the amount (breathing parameter) of gas inhaled into or exhaled from a lung of the subject. To test the pulmonary function, the diagnostic spirometer measures vital capacity by electrically directly integrating an output from an airflow converter connected to a mouth of a patient after covering a nose of the patient.

In this case, diagnostic variables to be obtained by integrating the output includes forced vital capacity (FVC), forced expiratory volume in Isec (FEV), a peak expiratory air flow rate (PEF), a forced mid-expiratory air flow rate (FET), a ratio of FEV/FVC, etc.

The existing diagnostic spirometer measures the vital capacity by measuring flow and volume of air based on mouth breathing while covering the nose of the subject. Such a method is noninvasive measurement, but there is a limit that it is impossible to know how much air passed through a mouth is divided into each of lungs

Further, there is a limit that it is impossible to know how air inhaled into each lung of the subject is distributed inside the lung.

In other words, a conventional spirometer measures global volume and global flow of air at a mouth of a subject and does not provide information about local volume and local flow at a certain area inside a lung, thereby having a problem that it is difficult to make a more accurate diagnosis with regard to a pulmonary function of a subject.

Besides, simple spirometry needs to measure change in the amount of air between the maximum inspiration and expiration, but it is difficult for the old and weak and some subjects to make the maximum inspiration and expiration. Therefore, there is a problem that accuracy in measurement and analysis is lowered.

[DISCLOSURE]

[TECHNICAL PROBLEM]

The disclosure is to provide a method and apparatus for a pulmonary function test, in which a conventional spirometer and electrical impedance tomography (EIT) are used together to collectively process and analyze a global variable obtained at a mouth of a subject and a local variable obtained from an image of an interior of a lung, thereby proposing spirotomometry.

Further, the disclosure is to provide a method and apparatus for a pulmonary function test, in which airflow mechanism information about an interior of a lung of a subject is provided based on spirotomometry, thereby improving usefulness of a vital capacity test.

Further, the disclosure is to provide a method and apparatus for a pulmonary function test, in which air distribution inside a lung is measured based on a thorax image by applying the EIT using electrodes attached to a thorax of a subject, and vital capacity is measured by electrically integrating an output of an airflow converter mounted to a mouth of the subject, thereby diagnosing a pulmonary function based on the measured air distribution inside the lung and the measured vital capacity.

Further, the disclosure is to provide a method and apparatus for a pulmonary function test, in which an integral value of real-time change in the amount of air due to respiration of a subject and a respiratory target value are displayed together on a monitor, and change in air distribution inside a lung is also measured based on an impedance image, thereby providing parameters about a lung volume and a pulmonary function without requiring a condition of the maximum inspiration and expiration.

[TECHNICAL SOLUTION]

An apparatus for a pulmonary function test includes a thorax electrode element provided with a plurality of electrodes for current injection and voltage detection, and attached along a chest circumference of a subject to be examined; a respiration measurer configured to receive a breathing parameter according to respiration of the subject; a controller configured to selectively supply currents to at least one pair of electrodes selected among the plurality of electrodes, and generate an image of an interior of a thorax based on impedance data by measuring voltage through unselected electrodes; a vital capacity calculator configured to calculate vital capacity of the subject based on the input breathing parameter; and a pulmonary function diagnoser configured to make a pulmonary function diagnosis of the subject in connection with a global variable obtained from the calculated vital capacity and a local variable obtained from the generated thorax image.

The controller may control a preset target respiratory setting value to be varied over time based on the global variable obtained from the calculated vital capacity, and the vital capacity calculator may obtain the breathing parameter of the subject and calculates the vital capacity of the subject with respect to the varied target respiratory setting value.

The vital capacity calculator may calculate the vital capacity of the subject according to volumes by integrating flow of air, based on the flow of air corresponding to the input breathing parameter.

The pulmonary function diagnoser may obtain the global variable according to the calculated vital capacity of the subject, and obtain the local variable by measuring at least one of air distribution change, degree and pattern inside a lung over time based on the generated thorax image.

Further, the pulmonary function diagnoser may make the pulmonary function diagnosis in connection with the local variable and the global variable.

The controller may include: a current injection module configured to inject currents having a plurality of frequency ranges through at least one pair of electrodes selected among the plurality of electrodes attached to the thorax of the subject; a voltage measuring module configured to measure voltage induced by the injected current from unselected electrodes among the plurality of electrodes; and an image generation module configured to generate an image of an interior of the thorax by measuring the impedance data in the thorax based on the measured voltage.

The current injection module may select the selected pair of electrodes and a frequency, generate and converts a voltage signal into a current corresponding to the selected frequency, and inject the converted current to the thorax of the subject through the selected pair of electrodes.

Further, the controller may further include a control module configured to control at least one pair of electrodes to be selected among the plurality of electrodes, control the unselected electrodes to be selected, and control the global variable to be obtained based on the breathing parameter output from the respiration measurer.

The plurality of electrodes may include at least one of a simple electrode or a complex electrode, and be arranged on one side of a base plate made of a flexible elastic material, and attached to the thorax.

The respiration measurer may be made of a disposable paper or plastic material, be shaped like a tube including an inlet to be in contact with a mouth of the subject and an outlet opposite to the inlet, and measure the breathing parameter according to the respiration of the subject from the inlet.

The apparatus for the pulmonary function test according to an embodiment of the disclosure may further include a display configured to display the global variable obtained in real time from the pulmonary function diagnoser, and a graph and image of local air distribution change inside a lung obtained from the thorax image.

A method of testing a pulmonary function, such as vital capacity of a subject, through an apparatus for a pulmonary function test include: calculating the vital capacity of the subject by measuring a breathing parameter according to respiration of the subject; selectively supplying currents to at least one pair of electrodes selected among a plurality of electrodes attached along a chest circumference of the subject, and generating an image of an interior of a thorax based on impedance data of voltage measured through unselected electrodes; and making a pulmonary function diagnosis of the subject in connection with a global variable obtained from the calculated vital capacity and a local variable obtained from the generated thorax image.

The calculation of the vital capacity may include calculating the vital capacity of the subject according to volumes by integrating flow of air, based on the flow of air corresponding to the measured breathing parameter.

The making of the pulmonary function diagnosis may include obtaining the global variable according to the calculated vital capacity of the subject, and obtaining the local variable by measuring at least one of air distribution change, degree and pattern inside a lung over time based on the generated thorax image.

Further, the making of the pulmonary function diagnosis may include making the pulmonary function diagnosis in connection with the local variable and the global variable.

[ADVANTAGEOUS EFFECTS]

According to an embodiment of the disclosure, a conventional spirometer and electrical impedance tomography (EIT) are used together to collectively process and analyze a global variable obtained at a mouth of a subject and a local variable obtained from an image of an interior of a lung, thereby proposing spirotomometry.

Further, according to an embodiment of the disclosure, airflow mechanism information about an interior of a lung of a subject is provided based on spirotomometry, thereby improving usefulness of a vital capacity test.

Further, according to an embodiment of the disclosure, real-time relative change in air distribution inside a lung is measured based on a thorax image by applying the EIT using electrodes attached to a thorax of a subject, and vital capacity is measured by electrically integrating an output of an airflow converter mounted to a mouth of the subject, thereby diagnosing a pulmonary function based on the measured air distribution inside the lung and the measured vital capacity.

Further, according to an embodiment of the disclosure, an integral value of real-time change in the amount of air due to respiration of a subject and a respiratory target value are displayed together on a monitor, and change in air distribution inside a lung is also measured based on an impedance image, thereby providing parameters about a lung volume and a pulmonary function without requiring a condition of the maximum inspiration and expiration.

Further, according to an embodiment of the disclosure, it is possible to analyze bronchial and pulmonary functions based on global respiratory variables obtained at an oral cavity among respiratory organs including the oral cavity, a bronchus and a lung, and respiratory indexes obtained inside a lung.

Further, according to an embodiment of the disclosure, it is possible to improve accuracy of a pulmonary function test, which has been lowered because some subjects such as the old and weak cannot make the maximum inspiration and expiration.

[DESCRIPTION OF DRAWINGS]

FIG. 1 is a block diagram of an apparatus for a pulmonary function test according to an embodiment of the disclosure.

FIG. 2A illustrates the apparatus for the pulmonary function test according to an embodiment of the disclosure, and FIG. 2B schematically illustrates a thorax electrode element.

FIGS. 3A and 3B schematically illustrate a complex electrode employed in the apparatus for the pulmonary function test shown in FIG. 2B.

FIGS. 4A to 4C schematically illustrate an electrode belt, and FIG. 4D illustrates an example that the electrode belt is attached to a body of a subject.

FIG. 5 shows a pulmonary respiration curve with breathing parameters.

FIG. 6A illustrates a breathing quantity measurement value calculated based on a breathing parameter of the subject, and FIG. 6B illustrates impedance data and thorax images measured at a thorax of a subject.

FIG. 7A illustrates a vital capacity measurement value in a section, and FIG. 7B illustrates an impedance image in a section.

FIG. 8 is a flowchart showing a method of testing a pulmonary function such as vital capacity of a subject through the apparatus for the pulmonary function test according to an embodiment of the disclosure.

[DETAILED DESCRIPTION OF THE INVENTION]

Below, embodiments of the disclosure will be described with reference to accompanying drawings and content shown the accompanying drawings without limiting the disclosure to the embodiments.

The terminology used herein is for the purpose of describing embodiments only and is not intended to be limiting of the disclosure. As used herein, the singular forms are intended to include the plural forms as well, unless the context clearly indicates otherwise. It will be further understood that the terms "comprises" and/or "comprising" when used herein specify the presence of stated elements, steps, operations, and/or components, but do not preclude the presence or addition of one or more other elements, steps, operations, and/or components.

A certain aspect or design disclosed in "embodiment," "example," "facet," "illustration," etc. used herein should not be construed to be better or more advantageous than other aspects or designs.

Further, terms 'or' are intended to indicate 'inclusive or' rather than 'exclusive or'. In other words, unless mentioned otherwise or the context clearly indicates otherwise, 'x employs a or b' is intended to mean any of the natural inclusive permutations.

Further, articles "a" or "an" as used in the present specification and claims should generally be construed to mean "one or more" unless specified otherwise or clear from context to be directed to a singular form.

Further, it will be understood that, although the terms first, second, etc. may be used herein to describe various elements, these elements should not be limited by these terms. These terms are only used to distinguish one element from another.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined here.

Meanwhile, in the following description, well-known functions or configurations will not be described in detail because they may obscure the gist of the disclosure. Further, the terminologies to be described below are defined in consideration of the functions within the scope of the disclosure and may vary depending on a user's or operator's intention or practice. Accordingly, some definitions are implied based on the content throughout the specification.

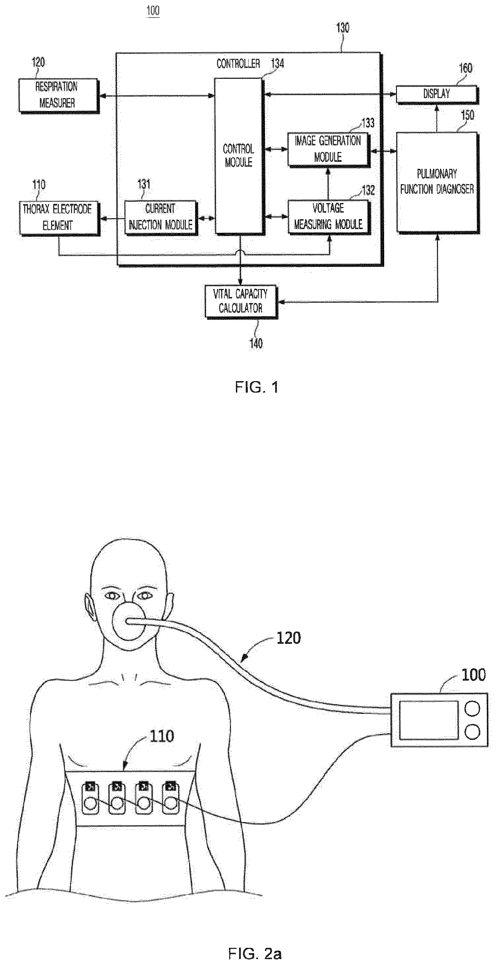

FIG. 1 is a block diagram of an apparatus for a pulmonary function test according to an embodiment of the disclosure.

Referring to FIG. 1, an apparatus 100 for a pulmonary function test according to an embodiment of the disclosure generates an image based on impedance data measured from a thorax electrode attached to a chest circumference of a subject, and diagnoses a pulmonary function by calculating vital capacity based on a breathing parameter of the subject.

To this end, the apparatus 100 for the pulmonary function test according to an embodiment of the disclosure includes a thorax electrode element 110, a respiration measurer 120, a controller 130, a vital capacity calculator 140, and a pulmonary function diagnoser 150.

The apparatus 100 for the pulmonary function test according to an embodiment of the disclosure may further include a display 160 configured to output an image and a signal in real time for a bio feedback.

The thorax electrode element 110 is provided with a plurality of electrodes for injecting an electric current and sensing voltage, and attached along a thorax circumference of a subject to be examined.

The plurality of electrodes may be at least one of a simple electrode and a complex electrode, and may be arrayed on one side of a base plate made of a flexible elastic material and attached to the thorax circumference.

Further, the plurality of electrodes may be EIT electrodes which are attached along the thorax of the subject and measure impedance data of an interior of a lung.

The EIT electrode is used to inject a low current, which cannot be recognized by a subject, for example, a highfrequency current of 1mA or below, and measure induced voltage. Current-voltage data measured by the EIT electrode may be used to detect a pattern through an imaging algorithm when a thorax (lung) is obstructed and a structural change that causes the obstruction.

The respiration measurer 120 receives the breathing parameter based on the respiration of the subject.

For example, the respiration measurer 120 is made of a disposable paper or plastic material, and shaped like a tube which includes an inlet to be in contact with a mouth of a subject and an outlet opposite to the inlet, thereby measuring the breathing parameter based on the respiration of the subject.

In more detail, the respiration measurer 120 may measure the breathing parameter based on the respiration of the subject from the existing spirometer, and the breathing parameter from the spirometer may be sampled almost simultaneously or simultaneously with reading of an impedance image. The breathing parameter is generally measured as synchronized.

The breathing parameter may be related to measurement of at least one among a respiration rate of a subject, a respiratory pressure of the subject, a respiratory airflow of the subject, end-expiration $CO_2$ of the subject, hypoglossal CO2 of the subject, and respiratory strength of the subject.

According to an embodiment, the breathing parameter may be related to measurement of at least one among a pattern of a respiratory curve of a subject, change in the respiratory curve of the subject, a respiratory curve based on inspiratory capacity of the subject, a respiratory curve based on expiratory capacity of the subject, a respiratory curve based on inspiratory pressure of the subject, a respiratory curve based on expiratory pressure of the subject, a respiratory curve based on inspiratory airflow of the subject, a respiratory curve based on expiratory airflow of the subject, change in a respiratory interval of the subject, and combination thereof.

The controller 130 selectively applies an electric current to at least one pair of electrodes selected among the plurality of electrodes, measures voltage through unselected electrodes, and makes an image of the interior of the thorax from impedance data.

To this end, the controller 130 according to an embodiment of the disclosure may include a current injection module 131, a voltage measuring module 132, an image generation module 133, and a control module 134.

The current injection module 131 may be configured to inject the current having a plurality of frequency ranges through at least one pair of electrodes selected among the plurality of electrodes attached to the thorax of the subject.

According to an embodiment, the current injection module 131 may be configured to select a pair of electrodes and a frequency, generate a voltage signal based on the selected frequency, convert the voltage into an electric current, and inject the converted current to the thorax of the subject through the selected pair of electrodes.

According to an alternative embodiment, the current injection module 131 may be configured to convert the voltage signal into two currents different in phase, correct the two currents to have the same amplitude and frequency, and inject the two currents to the thorax of the subject through the selected pair of electrodes.

The voltage measuring module 132 may be configured to measure voltages induced by the currents injected through the unselected electrodes among the plurality of electrodes.

For example, the voltage measuring module 132 may be configured to remove noise included in detected voltage based on the slope of the measured voltage, and replace a voltage corresponding to a section, a level of which is greater than a preset threshold, by a preset voltage when the slope of the detected voltage is higher than the threshold.

The image generation module 133 may be configured to measure the impedance data at the thorax based on the measured voltage, and generate an image of an interior of the thorax.

For example, the image generation module 133 may be configured to obtain a difference signal of the voltage induced by the injected current through the unselected electrodes among the plurality of electrodes, and obtain the impedance data according to the chest circumference of the subject and the position of the electrode.

Then, the image generation module 133 may be configured to generate the thorax image by restoring conductivity and permittivity images with regard to the thorax of the subject with the measured thorax impedance data.

Specifically, the image generation module 133 may be configured to have measurement protocols, which the plurality of electrodes have, to make images the thorax of the subject, thereby controlling sensitivity and resolutions according to the positions of the thorax electrode element 110. With measurement values using such a plurality of measurement protocols or combination thereof, and a sensitivity matrix improved in a three-dimensional (3D) modeling image generated through the thorax electrode element 110, conductivity and permittivity images corresponding to the upper airway of the subject and the interior of the thorax, i.e. the thorax image may be generated as the impedance images.

The control module 134 may be configured to control selection of at least one pair of electrodes from the plurality of electrodes, control selection of the unselected electrodes, and control obtainment of a global variable based on the breathing parameter output from the respiration measurer 120.

The control module 134 may be configured to control the current injection module 131 to measure the impedance data with regard to the thorax of the subject, and control the vital capacity calculator 140 to calculate the vital capacity of the subject based on the breathing parameter input according to the respiration of the subject.

For example, the control module 134 of the controller 130 may control the current injection module 131 and the voltage measuring module 132 for measuring the impedance image to obtain at least among impedance data, image information and time-difference information in synchronization with a feature point of a preset respiratory target-value graph for measuring the vital capacity and certain change in a calculation value of the vital capacity calculator 140.

According to an embodiment, the controller 130 may control the display 160 to display a graph of a preset target respiratory setting value varied depending on the global variable obtained in the vital capacity of the subject, which is calculated from the vital capacity calculator 140, and the subject may breathe according to the displayed target respiratory value.

Then, the respiration measurer 120 may obtain the breathing parameter of the subject, and the vital capacity calculator 140 may calculate real-time vital capacity by obtaining the breathing parameter of the subject with respect to the varied target respiratory setting value.

Alternatively, the control module 134 of the controller 130 may control the display 160 to display a real-time image and signal for a bio feedback, and guide the breathing quantity of subject to match the target value by outputting the respiratory target value differently over time

Further, the control module 134 may control at least one module of the voltage measuring module 132 and the image generation module 133 so as to measure vertical and horizontal impedance with regard to the thorax of the subject.

In obtaining the thorax image of the subject with the apparatus 100 for the pulmonary function test according to an embodiment of the disclosure, the EIT method is as follows.

The controller 130 according to an embodiment of the disclosure selects channel and sinusoidal frequencies in response to a command, and selects a pair of electrodes in the thorax electrode element 110 corresponding to the selected channel. The selected pair of electrodes are used for injecting the current to the upper airway corresponding to the thorax of the subject, and the unselected electrodes are used for measuring the voltage on the subject.

When the channel and sinusoidal frequencies are selected, the controller 130 outputs a control signal for controlling a field programmable gate array (FPGA, not shown). The control signal may include information about the selected frequencies.

The FPGA is configured to receive and store the control signal, and generate a sinusoidal voltage signal based on the received control signal. In particular, the FPGA is configured to generate a voltage signal based on frequency information included in the control signal, and transmits the generated voltage signal to two 16bit D/A converters (not shown). In this case, the FPGA controls a 8bit D/A converter (not shown) to adjust the amplitude of the voltage signal transmitted to the 16bit D/A converter. Then, the voltage signals output to the two 16bit D/A converters are converted into electric currents by the voltage-the current converters (not shown), and the two currents are transmitted to a corrector (not shown). The corrector (not shown) adjusts the two currents to have the same amplitude and frequency. Here, there is a phase difference of 180° between the two currents.

In addition, the control module 134 of the controller 130 injects the current into the thorax of the subject through the current injection module 131 so that two currents passed through the corrector can be transmitted to the pair of electrodes selected in the thorax electrode element 110.

The currents injected to the chest circumference of the subject causes the surface thereof to induce voltages different in level according to resistivity or conductivity of internal tissues. When the electrodes unselected in the thorax electrode element 110 sense the voltages on the surface of the chest circumference of the subject, the voltage measuring module 132 receives the voltages on the surfaces corresponding to the unselected electrodes.

Then, the voltage measuring module 132 determines whether the surface voltage data includes noise based on the slope of the sensed surface voltage data, and then replaces the corresponding voltage data by another voltage level when the surface voltage data includes the noise. Further, the control module 134 adjusts a gain of the voltage amplifier (not shown) according to the maximum level of the voltage data. For example, the control module 134 does not adjust the gain of the voltage amplifier when the maximum level of the voltage data reaches 90% of the maximum output of the A/D converter (not shown), but increases the gain of the voltage amplifier when the maximum level of the voltage data does not reach 90% of the maximum output of the A/D converter.

When the noise is removed from the voltage data and the gain of the voltage amplifier is adjusted, the voltage measuring module 132 amplifies the voltage data according to the adjusted gain and the A/D converter converts the voltage data into a digital value.

Then, the image generation module 133 processes the voltage data in consideration of gain information according to channels, based on the channel information and the gain information. When the detected voltage data different in gain is directly used, it is difficult to accurately represent electric characteristics inside the thorax of the subject. Therefore, the corresponding voltage level has to be decreased or increased according to the gain. For example, when a gain value is greater than a reference gain value, the corresponding voltage level is decreased and multiplied by a ratio of the gain value to the reference gain value.

Thus, the image generation module 133 may be configured to process the voltage data in consideration of the gain information according to the channels, and then obtain the impedance data based on the voltage data.

Then, the image generation module 133 generates an image of the interior of the thorax of the subject based on the impedance data. Alternatively, various methods of generating an image of an inside of a measurement target (a thorax) based on the voltage data on the surface of the thorax of the subject.

Further, the image generation module 133 may use an optical imaging device and a length measuring device to obtain outer appearance information based on markers positioned at the electrodes so as to form a 3D restoration model according to the shapes of the chest of the subject, and thereby providing a restoration algorithm.

Here, the restoration algorithm refers to an algorithm for restoring a 3D image, and employs a generally used algorithm.

Referring back to FIG. 1, the vital capacity calculator 140 of the apparatus 100 for the pulmonary function test according to an embodiment of the disclosure calculates the vital capacity of the subject based on an input breathing parameter.

For example, the vital capacity calculator 140 may calculate the vital capacity of the subject according to volumes by integrating flow of air corresponding to the respiration based on the input breathing parameter.

According to an embodiment, the vital capacity calculator 140 may calculate a respiratory volume, a respiratory flow, etc. based on the breathing parameter, and calculate the vital capacity of the subject from the calculated respiratory volume and respiratory flow. To calculate the vital capacity, publicly-known general-purpose calculation methods may be employed.

Further, the vital capacity calculator 140 may calculate at least one of a peak expiratory flow rate (PEF), forced expiratory volume in one second (FEV 1.0), forced vital capacity vital capacity (FVC), and FEV 1.0/FVC.

Here, the FVC refers to the amount of air exhaled quickly and strongly as much as possible after a subject inhales slowly and deeply as much as possible in a stable condition, and the FEV 1.0 refers to the amount of air forcedly exhaled for 1 second after a subject starts the maximum forced inspiration (i.e. inhales as much as possible).

Further, the FEV 1.0/FVC (or a ratio of FEV 1.0 to FVC 1) refers to an index useful for checking whether an airway is obstructed, and is also called "forced expiratory volume in 1 second." A normal person can exhale more than 70% of his/her own FCV in 1 second, and therefore FEV 1.0/FVC of the normal person is equal to or higher than 70%.

Based on the respiration guided to make the breathing quantity of the subject match the target value through the controller 130, the vital capacity calculator 140 may calculate a vital capacity index through change in global breathing quantity corresponding to change in guided vital capacity, change in air distribution according to areas inside a lung, and a time response corresponding to breathing efforts for matching the target value without requiring the maximum inspiration and expiration of the subject.

The pulmonary function diagnoser 150 diagnoses the pulmonary function of the subject in connection with the global variable obtained from the calculated vital capacity and the local variable obtained from the generated thorax image.

For example, the pulmonary function diagnoser 150 may obtain the global variable based on the calculated vital capacity of the subject, and obtain the local variable by measuring at least one of air distribution change, degree and pattern inside a lung over time based on the generated thorax image.

Then, the pulmonary function diagnoser 150 may diagnose the pulmonary function of the subject in connection with the local variable and the global variable.

In more detail, the pulmonary function diagnoser 150 may obtain the global variables about whether an airway is obstructed, how much the airway is obstructed, the lung volume, etc. according to the calculated vital capacity of the subject, and obtain the local variables by measuring at least one of air distribution change, degree and pattern inside a lung based on the thorax image. Thus, the pulmonary function diagnoser 150 may output diagnostic information about the pulmonary function such as a pulmonary diffusing image in the lung through collective process and analysis in connection with the global variable and the local variable.

Further, the pulmonary function diagnoser 150 may make a pulmonary function diagnosis, such as asthmatic, chronic obstructive pulmonary disease, etc. through a reaction test using bronchoconstrictor, bronchodilator, etc. based on time-difference analysis of change in a global breathing quantity variable and air distribution inside a lung.

The apparatus 100 for the pulmonary function test according to an embodiment of the disclosure may further include the display 160 to output a real-time image and signal for a bio feedback.

The display 160 may be configured to display a real-time global variable obtained from the pulmonary function diagnoser 150, a graph and image corresponding to change in air according to areas inside a lung obtained from the thorax image.

According to an embodiment, the display 160 may display a signal and image in real time to guide a subject to match the breathing quantity with the target value, and may alternatively output at least one of a warning signal, an alarm, a voice and image variation.

According to an alternative embodiment, the display 160 may display an image, a waveform, and a numerical value based on change in global breathing quantity according to respiration of a subject, change in air distribution according to areas inside a lung based on the generated thorax image, and effort time taken to match the breathing with the target value.

According to an alternative embodiment, the display 160 may include a touch screen and a button to have a user interface to receive at least one of a touch input and a button input corresponding to selection of a user (a subject or a manager).

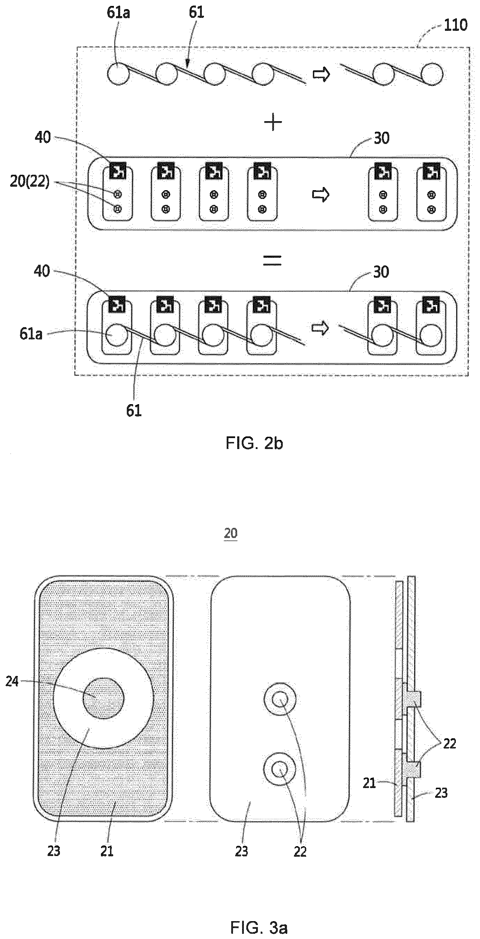

FIG. 2A illustrates the apparatus for the pulmonary function test according to an embodiment of the disclosure, and FIG. 2B schematically illustrates a thorax electrode element.

Referring to FIG. 2A, the apparatus 100 for the pulmonary function test according to an embodiment of the disclosure includes a thorax electrode element 110 attachable to a thorax of a subject, and a respiration measurer 120 to which the breathing parameter corresponding to the respiration of the subject is input.

The thorax electrode element 110 attached to the chest circumference of the subject is formed on a base plate at regular intervals, and measures impedance from a thorax image according the respiration of the subject as attached to the thorax of the subject.

According to an embodiment, the plurality of electrodes on the thorax electrode element 110 may include conductive fiber electrodes manufactured based on silver (Ag)-plated elastic fiber or polymer nanofiber web (PVDF), but be not limited thereto. Alternatively, the electrodes may be made of various materials having less reaction with skin even against long-time measurement.

According to an alternative embodiment, the base plate formed with the thorax electrode element 110 according to an embodiment of the disclosure is not necessarily limited to the shape of the belt-type array electrode. Alternatively, the base plate may include the thorax electrode element 110 having an array of at least one pattern or structure among a vest type, a belt type, and a patch type, by taking contact strength for enhancing a data measurement level into account while minimizing pressure that the subject feels.

In FIG. 2A, a plurality of electrodes in the thorax electrode element 110 according to an embodiment of the disclosure may be formed on a base plate at regular intervals or may be arranged with various arrays and structures on the parts (the thorax) targeted to be examined according to the features and purposes. Further, the base plate may have a certain length and area in order to measure impedance while surrounding the parts targeted to be examined, such as the thorax or the abdomen of the subject, but there are no limits to the length and the area. Alternatively, the length and the area may be varied depending on embodiments.

Further, the thorax electrode element 110 according to an embodiment of the disclosure may effectively measure distribution of an electric field around the surfaces of the thorax based on change in an electrode array structure and a measurement structure in such a manner that the plurality of electrodes are arranged in the form of a 2D or 3D array as attached to the thorax of the subject.

According to an embodiment, the thorax electrode element 110 to be formed on the base plate are arranged in the 3D array and configured to measure impedance corresponding to each layer, thereby leading to a more accurate and effective diagnosis than that of a conventional method of providing only a 2D cross-section image at a certain position.

In the apparatus 100 for the pulmonary function test according to an embodiment of the disclosure, the respiration measurer 120 includes a respiratory tube made of a disposable paper or plastic material, which may include a cylindrical tube having an inlet to be in contact with a mouth of a subject and an outlet opposite to the inlet.

The cylindrical tube may have a structure where the inlet to be in contact with a mouth of a subject and an outlet connected to the apparatus 100 for the pulmonary function test are connected.

In other words, the respiration measurer 120 may measure the breathing parameter according to the respiration of the subject through the cylindrical tube being in contact with the mouth of the subject, and the breathing parameter may be sampled almost simultaneously or simultaneously with reading of the impedance data of the thorax measured by the thorax electrode element 110 and the thorax image based on the impedance data. In general, the breathing parameter is measured as synchronized.

Further, the apparatus 100 for the pulmonary function test according to an embodiment of the disclosure may further include a sensing element (not shown) which is in contact with the parts of the subject targeted to be examined and senses a biometric signal.

The sensing element (not shown) may contact any body part of the subject targeted to be examined, and may be included in the thorax electrode element 110 according to embodiments.

According to an embodiment, the sensing element may include at least one of the posture sensor and the ECG sensor, and may be a fiber-based sensor to be attached to the body of the subject.

According to an alternative embodiment, the sensing element may sense an oxygen-saturation-in-blood (SpO2) signal through an optic sensor, which senses the amount of red light transmitting blood flowing in a peripheral blood vessel at a fingertip or a tip toe, so as to measure the oxygen saturation in blood of the subject.

For example, the sensing element may be provided in the form of a measurement terminal put on the fingertip, and may be actualized by an optic module that includes a red light-emitting diode (LED) of 660nm and an infrared LED of 940nm as a light emitter, and a photodiode and a phototransistor as a light receiver.

Referring back to FIG. 2B, the thorax electrode element 110 according to an embodiment of the disclosure include a plurality of electrodes 20, and is mountable along the chest circumference of the subject to be examined. To this end, the thorax electrode element 110 include the base plate 30 (hereinafter, referred to as an electrode belt) provided with the plurality of electrodes 20.

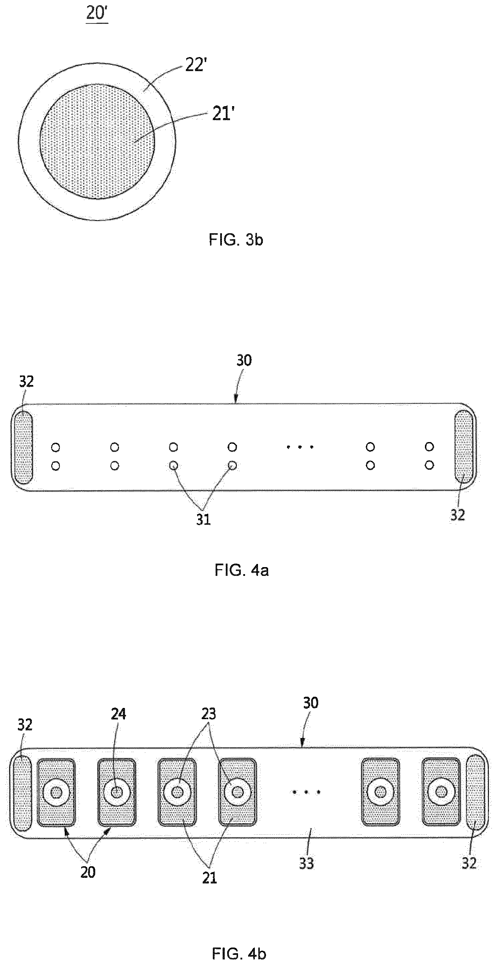

Below, the electrode belt with the plurality of electrodes 20 will be described in detail with reference to FIGS. 3A and 3B.

FIGS. 3A and 3B schematically illustrate a complex electrode employed in the apparatus for the pulmonary function test shown in FIG. 2B.

Referring back to FIG. 2B, a cable belt 61 is connected to a button-type connector 22 exposed through an electrode installation hole 31 in which the plurality of electrodes 20 (hereinafter, referred to as the complex electrode) is installed. In this case, the cable belt 61 may include a plurality of connection cable terminals 61a for injecting the currents, corresponding to the complex electrodes 20.

Thus, the voltage measuring module 132 in the apparatus 100 for the pulmonary function test according to an embodiment of the disclosure measures the voltage induced by the current injected to the complex electrode 20 through the cable belt 61. Specifically, the currents having multiple frequencies are generated through the cable belt 61 and supplied to the plurality of electrodes 20 of the electrode belt 30 put on the thorax of the subject while being subjected to amplitude and phase control. In this case, the currents having multiple frequencies are injected through a first electrode 21 of the complex electrode 20, and a voltage difference signal induced by the injected current is obtained through a second electrode 24 of the complex electrode 20.

Referring to FIG. 3A, the complex electrode 20 includes the first electrode 21 made of a conductive material for injecting a current, the second electrode 24 made of a conductive material for measuring a voltage, and a connector 22 shaped like a button to be connected to the cable belt 61. The first electrode 21 injects the current through a relatively large area as compared with the second electrode 24, and the second electrode 24 measures the voltage through a relatively small area as compared with the first electrode 21 and forms a pair with the second electrode 24 of the repetitive complex electrodes 20 on the cable belt 61.

In this case, the first electrode 21 is shaped like a flat plate, and the connector 22 shaped like a button is provided in the form of a pair of projections which protrude as being connected to the first electrode 21 and the second electrode 24. The first and second electrodes 21 and 24 of each of the plurality of complex electrodes 20 are installed with a nonconductor 23 made of a nonconductive material therebetween in the electrode belt 30.

The foregoing example illustrates that the plurality of electrodes 20 includes the complex electrode, but the plurality of electrodes 20 is not limited to this example. As an alternative example, the simple electrode 20' as shown in FIG. 3B is also possible. In a case of the simple electrode 20', the injection of the current or the measurement of the voltage is performed in a single conductive electrode 21' which is supported on a nonconductor 22'.

Further, the electrode 20 or 20' may be made of a flexible conductive fiber or a conductive polymeric material, and may be given in the form of a dry-type electrode.

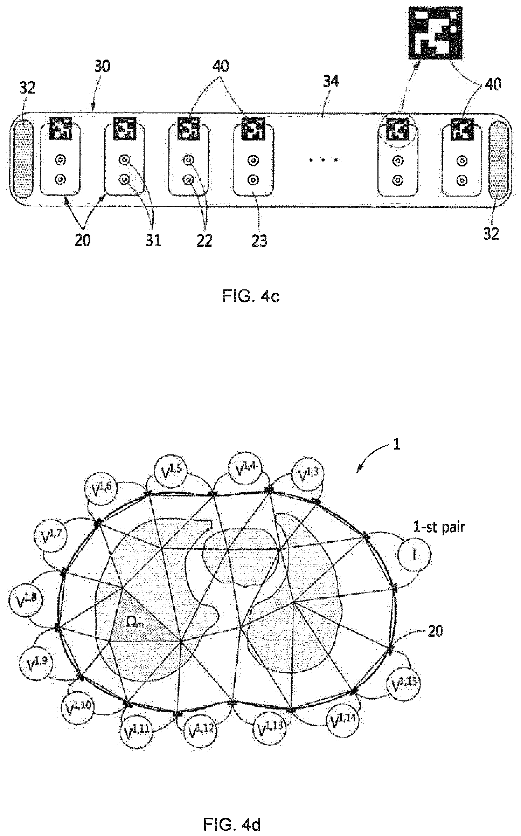

FIGS. 4A to 4C schematically illustrate an electrode belt, and FIG. 4D illustrates an example that the electrode belt is attached to a body of a subject.

Referring to FIG. 4A, the electrode belt 30 is made of an elastic material such as fiber, silicon and the like polymeric compound, and the number of provided electrode installation holes 31 and the number of installed complex electrodes 20 may be variable. Meanwhile, the electrode belt 30 is provided with a pair of fasteners 32 at opposite ends thereof, which are fastened to each other and maintain the electrode belt 30 as wound around the body of the subject.

This embodiment of the disclosure illustrates that the electrode belt 30 is wound around parts of the subject targeted to be examined, i.e. the chest circumference, and fastened by Velcro type fasteners 32 provided at the opposite ends thereof. Alternatively, without limitations, one of various fasteners such as hook types, etc. may be employed as the fasteners 32.

The electrode belt 30 includes a contact surface 33 to be in contact with the subject while having the plurality of complex electrodes 20 as shown in FIG. 4B, and an exposure surface 34 to be exposed to the controller 130 according to an embodiment of the disclosure while being opposite to the contact surface 33 as shown in FIG. 4C. In this case, the first electrode 21 and the second electrode 24 of the complex electrode 20 are exposed on the contact surface 33 of the electrode belt 30, and the button-type connector 22 connected to the cable belt 61 is exposed through the electrode installation hole 31 on the exposure surface 34. In addition, the exposure surface 34 of the electrode belt 30 includes a plurality of indicators 40, i.e. markers respectively corresponding to the plurality of complex electrodes 20 and having a plurality of colors and patterns corresponding to information of each of the plurality of complex electrodes 20.

According to an embodiment, the indicators 40, i.e. the markers may be different in shape according to the complex electrodes 20, and include corresponding channel numbers or pieces of data information different from one another, so that the position of the electrode can be identified as the indicator 40 is recognized (sensed).

Referring to FIG. 4D, the complex electrodes 20 included in the electrode belt are arranged in the form of the 3D array along the body circumference of the subject 1 to be examined, and it is thus possible to obtain a 3D image at a certain position (the thorax) because impedance corresponding to each layer is measurable by injecting the currents through the selected pair of electrodes and measuring the voltage induced by the injected currents.

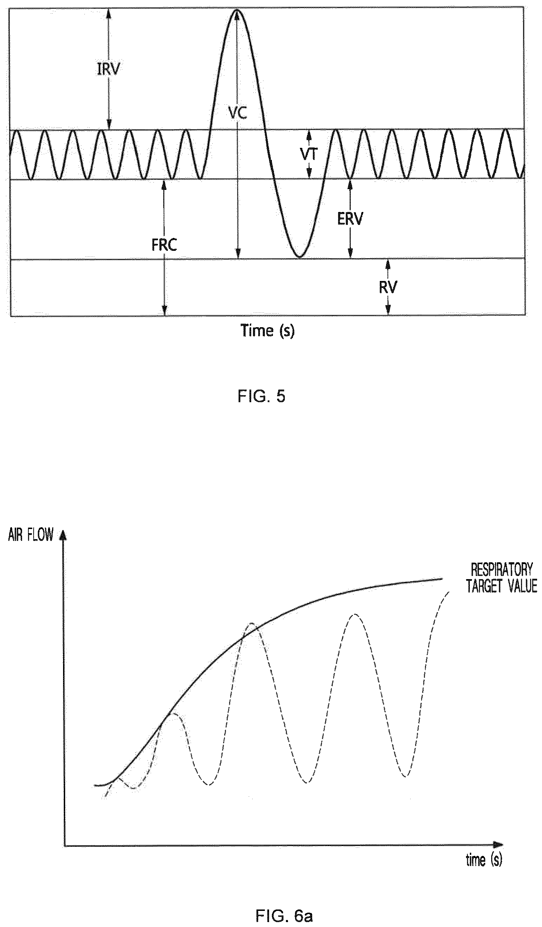

FIG. 5 shows a pulmonary respiration curve with breathing parameters.

In more detail, FIG. 5A shows the pulmonary respiration curve obtained from the breathing parameter according to the respiration of the subject.

Referring to FIG. 5, the apparatus for the pulmonary function test according to an embodiment of the disclosure may obtain the pulmonary respiration curve from the breathing parameter according to the respiration of the subject, and obtain various respiratory indexes from the obtained pulmonary respiration curve.

In more detail, the apparatus for the pulmonary function test according to an embodiment of the disclosure may obtain functional residual capacity (FRC), vital capacity (VC), expiratory reserve volume (ERV), and residual volume (RV) from the pulmonary respiration curve.

The FRC refers to volume of air remaining in a lung after normal expiration, the VC refers to the maximum volume of air exhaled at the maximum expiration after the maximum inspiration, the ERV refers to additional volume of air exhaled at the maximum forced expiration after normal expiration, and the RV refers to the minimum volume of air remaining in the lung after the maximum expiration.

The VC may be calculated including inspiratory reserve volume (IRV), tidal volume (VT), and the ERV.

The IRV refers to additional volume of air inhaled as much as possible, the VT refers to volume of air inhaled and exhaled corresponding to a breath, and the ERV refers to additional volume of air exhaled at the maximum forced expiration after normal expiration.

In other words, the apparatus for the pulmonary function test according to an embodiment of the disclosure may obtain various respiratory indexes including the vital capacity of the subject from the pulmonary respiration curve based on the breathing parameter.

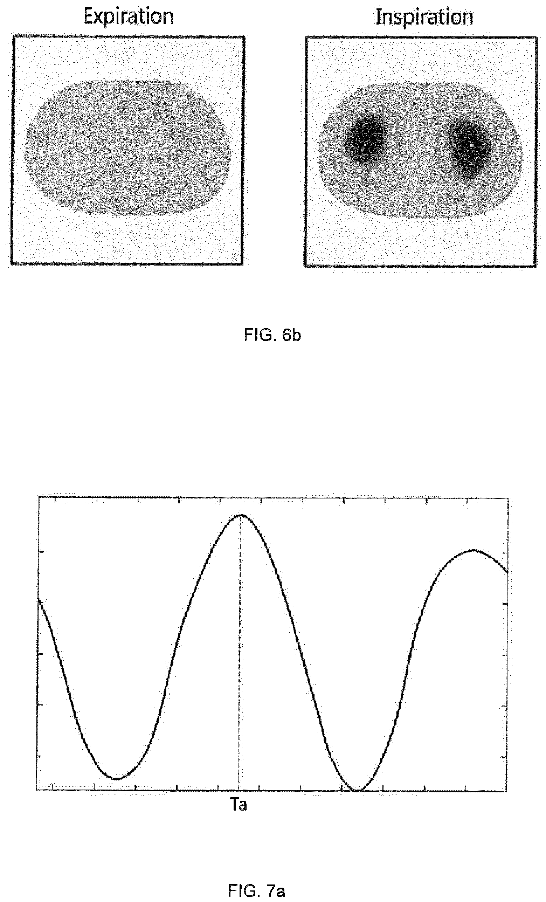

FIG. 6A illustrates a breathing quantity measurement value calculated based on a breathing parameter of the subject, and FIG. 6B illustrates impedance data and thorax images measured at a thorax of a subject.

Referring to FIG. 6A, the apparatus for the pulmonary function test according to an embodiment of the disclosure may calculate the vital capacity of the subject based on the breathing parameter measured by the respiration measurer according to the respiration of the subject, and obtain corresponding change in the global air volume.

Thus, the apparatus for the pulmonary function test according to an embodiment of the disclosure compares the breathing quantity measurement value corresponding to obtained change in the global air volume of the subject and the preset target respiratory setting value (or respiratory target value), and guides the breathing quantity of the subject to reach the respiratory target value.

The respiratory target value refers to not the maximum inspiration and expiration of the subject but change in a preset amount of air. The preset amount of air may refer to general average breathing quantity, or may be set by a subject.

Referring to FIG. 6B, it is possible to check air distribution according to areas with regard to the global vital capacity and the expiration and inspiration according to the air distribution inside the lung through the thorax image based on the impedance data measured by the electrodes attached along the chest circumference of the subject.

Thus, the apparatus for the pulmonary function test according to an embodiment of the disclosure obtains a vital capacity index through change in global breathing quantity corresponding to change in guided vital capacity, change in air distribution according to areas inside a lung, and a time response corresponding to breathing efforts for matching the target value without requiring the maximum inspiration and expiration of the subject.

Referring to FIGS. 6A and 6B, the apparatus for the pulmonary function test according to an embodiment of the disclosure identifies the thorax image inside the lung based on the impedance data according to the voltage measured by the thorax electrode element, and measures the breathing quantity according to the measured breathing parameter, thereby more accurately diagnosing the pulmonary function based on the vital capacity of the subject.

Further, the apparatus for the pulmonary function test according to an embodiment of the disclosure may separate and analyze a pulmonary internal function and a bronchus function and make a pulmonary function diagnosis, such as asthmatic, chronic obstructive pulmonary disease, etc. through a reaction test using bronchoconstrictor, bronchodilator, etc.

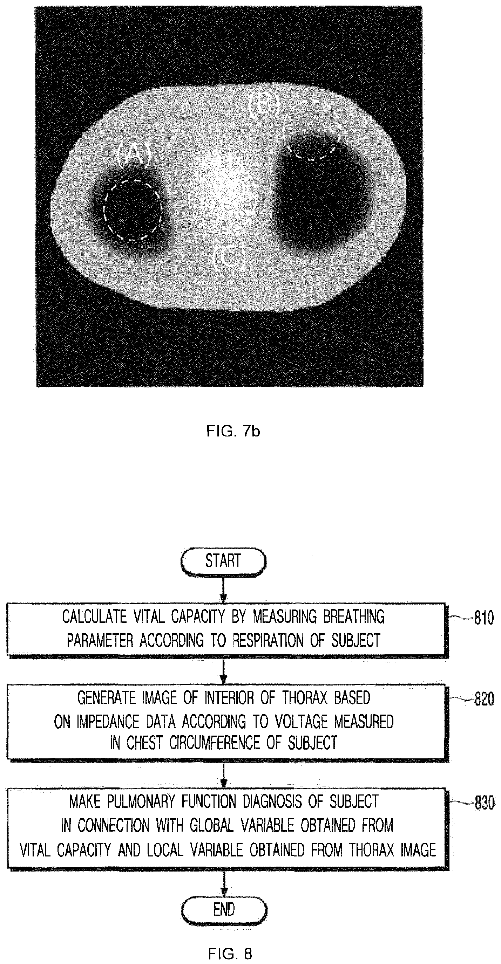

FIG. 7A illustrates a vital capacity measurement value in a section, and FIG. 7B illustrates an impedance image in a section.

In more detail, FIGS. 7A and 7B illustrate an example of change in air distribution according areas of a lung at time Ta according to change in global vital capacity measured at an oral cavity of a subject, by which a pulmonary diffusing capacity can be estimated together with a lung volume.

Further, FIGS. 7A and 7B illustrate the vital capacity measurement value and the impedance image, which are measured at the same time Ta.

Referring to FIG. 7A, the global vital capacity measurement value has the maximum vital capacity value at the section Ta.

FIG. 7B shows the thorax image at the section Ta corresponding to the maximum vital capacity value. Referring to FIG. 7B, it is possible to check a degree of inspiration air distribution according to areas by calculating a pixel value on the thorax image based on the impedance data.

For example, the apparatus for the pulmonary function test according to an embodiment of the disclosure may calculate a pixel value on the thorax image from a ratio of pixel impedance change to global impedance change, and the global vital capacity measurement value by the following [Expression 1].

[Expression 1]

$$\text{pixel value } (x) = \frac{pixel\ impedance\ change}{global\ impedance\ change} \times global\ vital\ capacity$$

With this, the apparatus for the pulmonary function test according to an embodiment of the disclosure can calculate pixel values according to areas (A), (B) and (C) on the thorax image, and obtain a distribution degree of global inspiration air according to the areas over time based on the calculated pixel values.

The pixel values of the areas (A), (B) and (C) may be varied and provided in real time depending on the local air distribution change inside the lung, and a degree of air distribution change may be displayed with numerical values such as 1%, 3%, 5%, 7%, 10%, etc.

FIG. 8 is a flowchart showing a method of testing a pulmonary function such as vital capacity of a subject through the apparatus for the pulmonary function test according to an embodiment of the disclosure.

Referring to FIG. 8, at operation 810, the breathing parameter according to the respiration of the subject is measured to calculate the vital capacity of the subject.

The operation 810 may include operation of calculating the vital capacity of the subject according to volumes by integrating flow of air corresponding to the measured breathing parameter.

At operation 820 the currents are selectively supplied to at least one pair of electrodes selected among the plurality of electrodes attached along the chest circumference of the subject, and an image corresponding to the interior of the thorax is generated based on the impedance data of the voltage measured through the unselected electrodes.

The operation 820 may include operation of obtaining a difference signal of the voltage induced by the injected current through the unselected electrodes among the plurality of electrodes, and generating an image of the interior of the thorax by obtaining the impedance data according to the chest circumference of the subject and the position of the electrode.

At operation 830 the pulmonary function of the subject is diagnosed in connection with the global variable obtained from the calculated vital capacity and the local variable obtained from the generated thorax image.

The operation 830 may include operation of obtaining the global variable according to the calculated vital capacity of the subject, and obtaining the local variable by measuring at least one of air distribution change, degree and pattern inside a lung over time based on the generated thorax image.

The operation 830 may include operation of making a pulmonary function diagnosis in connection with the local variable and the global variable.

The method of testing a pulmonary function, such as vital capacity of subject, through the apparatus for the pulmonary function test according to an embodiment of the disclosure may further include operation (not shown) of guiding the subject to breath with varied target respiratory setting value.

The method according to the embodiments may be actualized in the form of program instructions to be implemented through various computing means, and recorded in a computer readable medium. The computer readable medium may include a program instruction, a data file, a data structure solely or in combination. The program instructions recorded in the medium may be specially designed and configured for the embodiments or may be available as publicly known to a person having an ordinary skill in the art of computer software. The computer readable medium may for example include a hard disk, a floppy disk, a magnetic tape and the like magnetic media; a CD-ROM, a DVD and the like optical media; a floptical disk and the like magnetooptical media; and a ROM, a RAM, a flash memory and the like hardware device specially configured to store and implement a program instruction. The program instruction may for example include not only a machine code produced by a compiler, but also a high-level language to be implemented by a computer through an interpreter or the like. The hardware device may be configured to operate as one or more software modules to carry out the operations of the embodiment, and vice versa.

Although few embodiments are described with restricted examples and drawings, various modifications and changes can be made in the embodiments by a person having an ordinary skill in the art. For example, suitable results may be achieved even if the described techniques are performed in a different order, and/or if components in a described system, architecture, device, or circuit are combined in a different manner and/or replaced or supplemented by other components or their equivalents.

Therefore, other realizations, other embodiments and equivalents to claims may also belong to the scope of appended claims.

## Claims

1. An apparatus for a pulmonary function test, comprising
   a thorax electrode element provided with a plurality of electrodes for current injection and voltage detection, and attached along a chest circumference of a subject to be examined;
   a respiration measurer configured to receive a breathing parameter according to respiration of the subject;
   a controller configured to selectively supply currents to at least one pair of electrodes selected among the plurality of electrodes, and generate an image of an interior of a thorax based on impedance data by measuring voltage through unselected electrodes;
   a vital capacity calculator configured to calculate vital capacity of the subject based on the input breathing parameter; and
   a pulmonary function diagnoser configured to make a pulmonary function diagnosis of the subject in connection with a global variable obtained from the calculated vital capacity and a local variable obtained from the generated thorax image.

2. The apparatus for the pulmonary function test according to claim 1, wherein:

   the controller controls a preset target respiratory setting value to be varied over time based on the global variable obtained from the calculated vital capacity, and
   the vital capacity calculator obtains the breathing parameter of the subject and calculates the vital capacity of the subject with respect to the varied target respiratory setting value.

3. The apparatus for the pulmonary function test according to claim 1, wherein the vital capacity calculator calculates the vital capacity of the subject according to volumes by integrating flow of air, based on the flow of air corresponding to the input breathing parameter.

4. The apparatus for the pulmonary function test according to claim 3, wherein the pulmonary function diagnoser obtains the global variable according to the calculated vital capacity of the subject, and obtains the local variable by measuring at least one of air distribution change, degree and pattern inside a lung over time based on the generated thorax image.

5. The apparatus for the pulmonary function test according to claim 4, wherein the pulmonary function diagnoser makes the pulmonary function diagnosis in connection with the local variable and the global variable.

6. The apparatus for the pulmonary function test according to claim 1, wherein the controller comprises:

   a current injection module configured to inject currents having a plurality of frequency ranges through at least one pair of electrodes selected among the plurality of electrodes attached to the thorax of the subject;
   a voltage measuring module configured to measure voltage induced by the injected current from unselected electrodes among the plurality of electrodes; and
an image generation module configured to generate an image of an interior of the thorax by measuring the impedance data in the thorax based on the measured voltage.

7.  The apparatus for the pulmonary function test according to claim 6, wherein the current injection module selects the selected pair of electrodes and a frequency, generates and converts a voltage signal into a current corresponding to the selected frequency, and injects the converted current to the thorax of the subject through the selected pair of electrodes.

8.  The apparatus for the pulmonary function test according to claim 5, wherein the controller further comprises a control module configured to control at least one pair of electrodes to be selected among the plurality of electrodes, control the unselected electrodes to be selected, and control the global variable to be obtained based on the breathing parameter output from the respiration measurer.

9.  The apparatus for the pulmonary function test according to claim 1, wherein the plurality of electrodes comprises at least one of a simple electrode or a complex electrode, and is arranged on one side of a base plate made of a flexible elastic material, and attached to the thorax.

10. The apparatus for the pulmonary function test according to claim 1, wherein the respiration measurer is made of a disposable paper or plastic material, is shaped like a tube comprising an inlet to be in contact with a mouth of the subject and an outlet opposite to the inlet, and measures the breathing parameter according to the respiration of the subject from the inlet.

11. The apparatus for the pulmonary function test according to claim 1, further comprising a display configured to display the global variable obtained in real time from the pulmonary function diagnoser, and a graph and image of local air distribution change inside a lung obtained from the thorax image.

12. A method of testing a pulmonary function, such as vital capacity of a subject, through an apparatus for a pulmonary function test, the method comprising:

   calculating the vital capacity of the subject by measuring a breathing parameter according to respiration of the subject;
   selectively supplying currents to at least one pair of electrodes selected among a plurality of electrodes attached along a chest circumference of the subject, and generating an image of an interior of a thorax based on impedance data of voltage measured through unselected electrodes; and
   making a pulmonary function diagnosis of the subject in connection with a global variable obtained from the calculated vital capacity and a local variable obtained from the generated thorax image.

13. The method according to claim 12, wherein the calculation of the vital capacity comprises calculating the vital capacity of the subject according to volumes by integrating flow of air, based on the flow of air corresponding to the measured breathing parameter.

14. The method according to claim 13, wherein the making of the pulmonary function diagnosis comprises obtaining the global variable according to the calculated vital capacity of the subject, and obtaining the local variable by measuring at least one of air distribution change, degree and pattern inside a lung over time based on the generated thorax image.

15. The method according to claim 14, wherein the making of the pulmonary function diagnosis comprises making the pulmonary function diagnosis in connection with the local variable and the global variable.

16. A computer program stored in a computer readable recording medium to perform the method according to any one of claims 12 to 15.

FIG. 1

FIG. 2a

FIG. 2b

20

FIG. 3a

20'

22'

21'

FIG. 3b

32

30

○ ○ ○ ○ • • • ○ ○

○ ○ ○ ○ ○ ○

31

32

FIG. 4a

32

24

23

30

20

21

33

32

FIG. 4b

16

FIG. 4c

FIG. 4d

FIG. 5

FIG. 6a

Expiration

Inspiration

FIG. 6b

Ta

FIG. 7a

FIG. 7b

START

CALCULATE VITAL CAPACITY BY MEASURING BREATHING
PARAMETER ACCORDING TO RESPIRATION OF SUBJECT —810

GENERATE IMAGE OF INTERIOR OF THORAX BASED
ON IMPEDANCE DATA ACCORDING TO VOLTAGE MEASURED
IN CHEST CIRCUMFERENCE OF SUBJECT —820

MAKE PULMONARY FUNCTION DIAGNOSIS OF SUBJECT
IN CONNECTION WITH GLOBAL VARIABLE OBTAINED FROM
VITAL CAPACITY AND LOCAL VARIABLE OBTAINED FROM THORAX IMAGE —830

END

FIG. 8

# EP 3 542 718 A1

## INTERNATIONAL SEARCH REPORT

| International application No. |
|---|
| PCT/KR2017/012920 |

| A. | CLASSIFICATION OF SUBJECT MATTER |
|---|---|

*A61B 5/091(2006.01)i, A61B 5/053(2006.01)i*

According to International Patent Classification (IPC) or to both national classification and IPC

| B. | FIELDS SEARCHED |
|---|---|

Minimum documentation searched (classification system followed by classification symbols)
A61B 5/091; A61B 5/05; A61B 5/085; A61N 1/378; A61B 5/087; A61B 10/00; A61B 5/053

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: pulmonary function, respiration, electrode, impedance, imaging

| C. | DOCUMENTS CONSIDERED TO BE RELEVANT | |
|---|---|---|
| Category* | Citation of document, with indication, where appropriate, of the relevant passages | Relevant to claim No. |
| A | KR 10-2013-0108273 A (RESPIRATORY MOTION, INC.) 02 October 2013 See paragraphs [27]-[59], [132]; claim 1; and figures 8, 9. | 1-16 |
| A | JP 2015-144860 A (CAREFUSION 207 INC.) 13 August 2015 See paragraphs [3], [12]-[20]; claim 1; and figure 1. | 1-16 |
| A | KR 10-2011-0115302 A (NUGA MEDICAL CO., LTD.) 21 October 2011 See paragraphs [21]-[25]; claim 1; and figure 1. | 1-16 |
| A | JP 2016-526466 A (PULMONE ADVANCED MEDICAL DEVICES LTD.) 05 September 2016 See paragraphs [88]-[90]; claim 1; and figure 3. | 1-16 |
| A | US 2012-0053654 A1 (TEHRANI et al.) 01 March 2012 See paragraphs [56]-[68]; claim 1; and figures 1, 2. | 1-16 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 02 FEBRUARY 2018 (02.02.2018) | **05 FEBRUARY 2018 (05.02.2018)** |
| Name and mailing address of the ISA/KR Korean Intellectual Property Office Government Complex-Daejeon, 189 Seonsa-ro, Daejeon 302-701, Republic of Korea Facsimile No. +82-42-481-8578 | Authorized officer Telephone No. |

Form PCT/ISA/210 (second sheet) (January 2015)

INTERNATIONAL SEARCH REPORT

Information on patent family members

International application No.

**PCT/KR2017/012920**

| Patent document cited in search report | Publication date | Patent family member | Publication date |
|---|---|---|---|
| KR 10-2013-0108273 A | 02/10/2013 | AU 2011-289159 A1 | 14/02/2013 |
| | | AU 2011-289159 B2 | 03/09/2015 |
| | | AU 2012-283905 A1 | 27/03/2014 |
| | | AU 2012-283905 B2 | 29/10/2015 |
| | | AU 2015-264875 A1 | 24/12/2015 |
| | | CA 02805124 A1 | 16/02/2012 |
| | | CA 02843806 A1 | 24/01/2013 |
| | | CA 02981390 A1 | 16/02/2012 |
| | | CL 2013000205 A1 | 23/08/2013 |
| | | CL 2014000146 A1 | 01/08/2014 |
| | | CN 103153184 A | 12/06/2013 |
| | | CN 103153184 B | 27/04/2016 |
| | | CN 103957862 A | 30/07/2014 |
| | | CN 103957862 B | 08/02/2017 |
| | | EP 2603138 A1 | 19/06/2013 |
| | | EP 2603138 B1 | 01/11/2017 |
| | | EP 2734170 A1 | 28/05/2014 |
| | | IL 223972 A | 28/02/2013 |
| | | IL 223973 A | 28/02/2013 |
| | | IL 230526 A | 31/03/2014 |
| | | JP 2014-502854 A | 06/02/2014 |
| | | JP 2014-524816 A | 25/09/2014 |
| | | JP 2016-179187 A | 13/10/2016 |
| | | JP 5922233 B2 | 24/05/2016 |
| | | JP 5944899 B2 | 05/07/2016 |
| | | KR 10-1805078 B1 | 10/01/2018 |
| | | KR 10-2014-0054103 A | 08/05/2014 |
| | | MX 2013001557 A | 28/06/2013 |
| | | MX 2014000761 A | 06/10/2014 |
| | | US 2012-0041279 A1 | 16/02/2012 |
| | | US 2013-0023781 A1 | 24/01/2013 |
| | | US 2016-0367186 A1 | 22/12/2016 |
| | | WO 2012-021900 A1 | 16/02/2012 |
| | | WO 2013-013153 A1 | 24/01/2013 |
| | | ZA 201300054 B | 25/09/2013 |
| | | ZA 201400506 B | 29/04/2015 |
| JP 2015-144860 A | 13/08/2015 | AU 2008-299098 A1 | 19/03/2009 |
| | | AU 2008-299098 B2 | 02/05/2013 |
| | | BR PI0817068 A2 | 11/10/2016 |
| | | CA 02699281 A1 | 19/03/2009 |
| | | CN 101801265 A | 11/08/2010 |
| | | CN 101801265 B | 24/07/2013 |
| | | EP 2194863 A1 | 16/06/2010 |
| | | EP 2194863 B1 | 30/03/2016 |
| | | JP 2010-538748 A | 16/12/2010 |
| | | JP 5793301 B2 | 14/10/2015 |
| | | JP 6043829 B2 | 14/12/2016 |
| | | NZ 584034 A | 28/10/2011 |

Form PCT/ISA/210 (patent family annex) (January 2015)

INTERNATIONAL SEARCH REPORT
Information on patent family members

| International application No. |
|---|
| **PCT/KR2017/012920** |

| Patent document cited in search report | Publication date | Patent family member | Publication date |
|---|---|---|---|
| | | RU 2010114175 A | 20/10/2011 |
| | | US 2009-0118634 A1 | 07/05/2009 |
| | | US 8808193 B2 | 19/08/2014 |
| | | WO 2009-035965 A1 | 19/03/2009 |
| | | ZA 201001732 B | 28/08/2013 |
| KR 10-2011-0115302 A | 21/10/2011 | KR 10-1159209 B1 | 25/06/2012 |
| | | WO 2011-129474 A1 | 20/10/2011 |
| JP 2016-526466 A | 05/09/2016 | CN 105722460 A | 29/06/2016 |
| | | EP 3019082 A1 | 18/05/2016 |
| | | US 2016-0106341 A1 | 21/04/2016 |
| | | WO 2015-005958 A1 | 15/01/2015 |
| US 2012-0053654 A1 | 01/03/2012 | CN 101305394 A | 12/11/2008 |
| | | CN 101305394 B | 10/10/2012 |
| | | CN 101345871 A | 14/01/2009 |
| | | CN 101345871 B | 04/01/2012 |
| | | EP 1955279 A2 | 13/08/2008 |
| | | EP 1955279 B1 | 17/05/2017 |
| | | TW 200838310 A | 16/09/2008 |
| | | TW 1478584 B | 21/03/2015 |
| | | US 2005-0085734 A1 | 21/04/2005 |
| | | US 2005-0085865 A1 | 21/04/2005 |
| | | US 2005-0085866 A1 | 21/04/2005 |
| | | US 2005-0085867 A1 | 21/04/2005 |
| | | US 2005-0085868 A1 | 21/04/2005 |
| | | US 2005-0085869 A1 | 21/04/2005 |
| | | US 2006-0030894 A1 | 09/02/2006 |
| | | US 2006-0036294 A1 | 16/02/2006 |
| | | US 2006-0122662 A1 | 08/06/2006 |
| | | US 2006-0142815 A1 | 29/06/2006 |
| | | US 2006-0149334 A1 | 06/07/2006 |
| | | US 2006-0155341 A1 | 13/07/2006 |
| | | US 2006-0167523 A1 | 27/07/2006 |
| | | US 2006-0247729 A1 | 02/11/2006 |
| | | US 2007-0021795 A1 | 25/01/2007 |
| | | US 2007-0107062 A1 | 10/05/2007 |
| | | US 2008-0154330 A1 | 26/06/2008 |
| | | US 2008-0161878 A1 | 03/07/2008 |
| | | US 2008-0167695 A1 | 10/07/2008 |
| | | US 2008-0177347 A1 | 24/07/2008 |
| | | US 2008-0183239 A1 | 31/07/2008 |
| | | US 2008-0183240 A1 | 31/07/2008 |
| | | US 2008-0188903 A1 | 07/08/2008 |
| | | US 2008-0188904 A1 | 07/08/2008 |
| | | US 2008-0208281 A1 | 28/08/2008 |
| | | US 2008-0215106 A1 | 04/09/2008 |
| | | US 2008-0219357 A1 | 11/09/2008 |
| | | US 2008-0288010 A1 | 20/11/2008 |

Form PCT/ISA/210 (patent family annex) (January 2015)

INTERNATIONAL SEARCH REPORT
Information on patent family members

International application No.

**PCT/KR2017/012920**

| Patent document cited in search report | Publication date | Patent family member | Publication date |
|---|---|---|---|
| | | US 2008-0288015 A1 | 20/11/2008 |
| | | US 2011-0230932 A1 | 22/09/2011 |
| | | US 2011-0288609 A1 | 24/11/2011 |
| | | US 2012-0109249 A1 | 03/05/2012 |
| | | US 2012-0158091 A1 | 21/06/2012 |
| | | US 2012-0323293 A1 | 20/12/2012 |
| | | US 2013-0197601 A1 | 01/08/2013 |
| | | US 2013-0296964 A1 | 07/11/2013 |
| | | US 2013-0296973 A1 | 07/11/2013 |
| | | US 2015-0034081 A1 | 05/02/2015 |
| | | US 2017-0036017 A1 | 09/02/2017 |
| | | US 7970475 B2 | 28/06/2011 |
| | | US 7979128 B2 | 12/07/2011 |
| | | US 8116872 B2 | 14/02/2012 |
| | | US 8140164 B2 | 20/03/2012 |
| | | US 8160711 B2 | 17/04/2012 |
| | | US 8200336 B2 | 12/06/2012 |
| | | US 8244358 B2 | 14/08/2012 |
| | | US 8255056 B2 | 28/08/2012 |
| | | US 8265759 B2 | 11/09/2012 |
| | | US 8280513 B2 | 02/10/2012 |
| | | US 8335567 B2 | 18/12/2012 |
| | | US 8348941 B2 | 08/01/2013 |
| | | US 8369398 B2 | 05/02/2013 |
| | | US 8412331 B2 | 02/04/2013 |
| | | US 8467876 B2 | 18/06/2013 |
| | | US 8509901 B2 | 13/08/2013 |
| | | US 8893302 B2 | 18/11/2014 |
| | | US 9259573 B2 | 16/02/2016 |
| | | US 9370657 B2 | 21/06/2016 |
| | | WO 2005-037077 A2 | 28/04/2005 |
| | | WO 2005-037077 A3 | 09/09/2005 |
| | | WO 2005-037172 A2 | 28/04/2005 |
| | | WO 2005-037172 A3 | 04/08/2005 |
| | | WO 2005-037173 A2 | 28/04/2005 |
| | | WO 2005-037173 A3 | 30/06/2005 |
| | | WO 2005-037174 A2 | 28/04/2005 |
| | | WO 2005-037174 A3 | 09/06/2005 |
| | | WO 2005-037220 A2 | 28/04/2005 |
| | | WO 2005-037220 A3 | 07/07/2005 |
| | | WO 2005-037366 A1 | 28/04/2005 |
| | | WO 2007-058938 A2 | 24/05/2007 |
| | | WO 2007-058938 A3 | 30/04/2009 |
| | | WO 2007-059377 A2 | 24/05/2007 |
| | | WO 2007-059377 A3 | 03/04/2008 |

Form PCT/ISA/210 (patent family annex) (January 2015)